# EUROPÄISCHE PATENTANMELDUNG

(11) **EP 0 983 986 A1**
(43) Veröffentlichungstag der Anmeldung: **08.03.2000**
(21) Anmeldenummer: 99116620.8
(22) Anmeldetag: 25.08.1999
(51) Int. Cl.: C07C 41/42, C07C 41/06, C07C 43/15

(54) **Verfahren zur Abtrennung von 1,3-Butadien und Wasser aus Stoffgemischen enthaltend 1,3-Butadien, 1-n-Butoxybuten-2, 3-n-Butoxybuten-1, n-Butanol, Di-n-butylether und Wasser**

(30) Priorität: 25.08.1998 DE 19838449
(71) Anmelder: BASF AKTIENGESELLSCHAFT, 67056 Ludwigshafen (DE)
(72) Erfinder: Aron, Maik, 67251 Freinsheim (DE)

(57) **Zusammenfassung**

Verfahren zur Abtrennung von 1,3-Butadien und Wasser aus Stoffgemischen enthaltend 1,3-Butadien, 1-n-Butoxybuten-2, 3-n-Butoxybuten-1, n-Butanol, Di-n-butylether und Wasser, die bei der säurekatalysierten Umsetzung von 1,3-Butadien mit n-Butanol anfallen, wobei die Abtrennung von 1,3-Butadien und Wasser durch Destillation erfolgt.

## Beschreibung

Die vorliegende Erfindung betrifft ein verbessertes Verfahren zur Abtrennung von 1,3-Butadien und Wasser aus Stoffgemischen, die bei der säurekatalysierten Umsetzung von 1,3-Butadien mit n-Butanol anfallen und 1,3-Butadien, 1-n-Butoxybuten-2, 3-n-Butoxybuten-1, n-Butanol, Di-n-butylether sowie Wasser enthalten.

In der EP-A 739 332 (D1) wird ein Verfahren zur Herstellung von n-Butyraldehyd und/oder n-Butanol auf Basis von 1,3-Butadien beschrieben, wonach 1,3-Butadien mit einem Alkohol der Formel I in Anwesenheit saurer Katalysatoren zu den Produkten 1-Alkoxybuten-2 II und 3-Alkoxybuten-1 III umgesetzt wird.

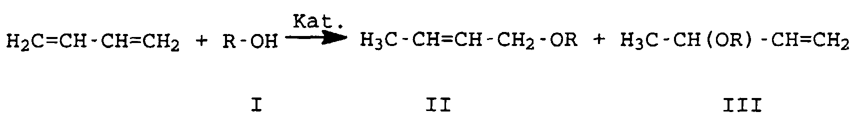

Wertprodukt dieser ersten Reaktionsstufe ist das 1-Alkoxybuten-2 II, das mit Hilfe eines weiteren Katalysators entweder mit Wasser zum n-Butyraldehyd IV oder mit Wasser/Wasserstoff zu n-Butanol V umgesetzt werden kann. Der gleichzeitig wieder freigewordene Alkohol I kann abgetrennt und erneut in die Butadien-Umsetzung der ersten Synthesestufe zurückgeführt werden.

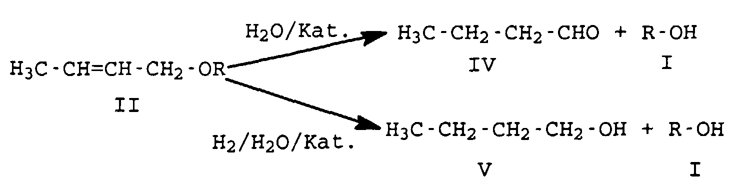

Das bei der Umsetzung von 1,3-Butadien mit dem Alkohol I als zweite Hauptkomponente gebildete 3-Alkoxybuten-1 III kann nicht wie das 1-Alkoxybuten-2 II direkt zu den Zielprodukten n-Butyraldehyd IV und/oder n-Butanol V umgesetzt werden. Es ist daher erforderlich, das Produkt III vor der Weiterverarbeitung aus dem Reaktionsgemisch der 1,3-Butadien/Alkohol I-Umsetzung abzutrennen und erneut in die 1,3-Butadien/Alkohol I-Umsetzung einzuschleusen, wo es gemäß der Lehre von D1 zum gewünschten 1-Alkoxybuten-2 II isomerisiert. Die Rückführung und Isomerisierung von 3-Alkoxybuten-1 III führt letztendlich zur Unterdrückung der Neubildung des unerwünschten Addukts III, so daß bei Anwendung dieser Kreislauffahrweise in der Gesamtbilanz dieses Kreisprozesses praktisch nur das gewünschte 1-Alkoxybuten-2 II gebildet wird.

Ein Syntheseverfahren ist besonders dann wirtschaftlich, wenn nicht umgesetzte Rohstoffe und wiederverwertbare Nebenprodukte erneut in den Syntheseprozeß eingeschleust sowie nicht im Prozeß verwertbare Nebenkomponenten aus dem Prozeß abgetrennt werden können.

Bezogen auf die erste Synthesestufe des in D1 beschriebenen Prozesses bedeutet dies, daß nicht umgesetztes 1,3-Butadien, nicht umgesetzter Alkohol I (sowohl Reaktant und Lösungsmittel) und 3-Alkoxybuten-1 III aus dem Reaktionsgemisch abgetrennt und wieder in die Synthesestufe zurückgeführt werden müssen.

Da Alkohole in Gegenwart saurer Katalysatoren unter Bedingungen, wie sie nach dem Stand der Technik (D1) auftreten, nach folgender Reaktionsgleichung in Spuren Dialkylether VI bilden, 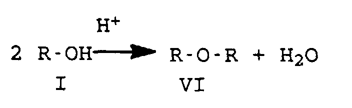 müssen, zur Vermeidung entsprechender störender Aufpegelungen, auch Dialkylether VI und Wasser aus dem Reaktionsgemisch entfernt werden.

Bei Verwendung von n-Butanol als Alkohol I liegen im Reaktionsgemisch der ersten Synthesestufe des in D1 beschriebenen Prozesses im wesentlichen die

Komponenten

| | |
|---|---|
| | 1,3-Butadien |
| | Wasser |
| I | n-Butanol |
| II | 1-n-Butoxybuten-2 |
| III | 3-n-Butoxybuten-1 |
| VI | Di-n-butylether |

vor.

Das in D1 dargestellte Verfahrenskonzept sieht in der ersten Synthesestufe vor, 1,3-Butadien mit n-Butanol und zurückgeführtem 3-n-Butoxybuten-1 mittels eines sauren Katalysators in einem Reaktor umzusetzen, den Reaktionsaustrag zu entspannen und das bei der Reaktion nicht vollständig umgesetzte und nach der Entspannung gasförmig vorliegende 1,3-Butadien in einem Gas-Flüssigkeits-Abscheider abzutrennen und in den Synthesereaktor zurückzuführen. Der verbleibende flüssige Reaktionsaustrag wird in einer Destillationskolonne aufgetrennt. Am Sumpf der Kolonne fallen 1-n-Butoxybuten-2 und Hochsieder an. Die am Kopf der Kolonne abgetrennten 3-n-Butoxybuten-1 und n-Butanol werden wieder in die Reaktionsstufe zurückgeführt. Das im Zulauf der Kolonne noch gelöste 1,3-Butadien fällt am Kopf der Kolonne an und muß vor Wiedereinsatz im Reaktor mit Kühlsole kondensiert oder aus dem Reaktionssystem ausgeschleust und einer Verbrennung zugeführt werden.

Als nachteilig hat sich bei dem genannten Verfahren herausgestellt, daß die 1,3-Butadien-Abtrennung mittels eines Gas-Flüssigskeits-Abscheiders nur unvollständig realisiert werden kann und noch nennenswerte Mengen an 1,3-Butadien in der Flüssigphase gelöst bleiben. Erfolgt die Entspannung des Reaktoraustrages im Gas-Flüssigkeits-Abscheider bei niedrigeren Drücken, wie beispielsweise kleiner als 3 bar, was aus Gründen der Restlöslichkeit des 1,3-Butadiens in der flüssigen Phase sinnvoll wäre, müßte das gasförmige 1,3-Butadien aufgrund des geringen Siedepunktes auch hier vor dem Wiedereinsatz mit einer Kühlsole anstelle von Flußwasser kondensiert werden. Die Kühlung mit Kühlsole ist apparativ aufwendig und verursacht daher in der Regel erhebliche Betriebs- und Energiekosten.

Da 1,3-Butadien als krebserregend eingestuft ist und insbesondere in reiner Form zu Polymerisation neigt, ist es außerdem vorteilhaft, die Anwesenheit von 1,3-Butadien auf möglichst wenig Apparate zu beschränken.

Weiterhin wird das unter Säurekatalyse neben dem Di-n-butylether gebildete Wasser bei der 3-n-Butoxybuten-1- und n-Butanol-Rückführung mitgeschleppt und reichert sich im Reaktionskreislauf der ersten Synthesestufe in unerwünschter Weise an.

Es stellte sich somit die Aufgabe, ein verbessertes Verfahren zu finden, das eine möglichst vollständige und wirtschaftliche Rückführung des nicht umgesetzten 1,3-Butadiens aus Reaktionsgemischen resultierend aus der säurekatalysierten Umsetzung von 1,3-Butadien mit n-Butanol gemäß D1 erlaubt. Der vorliegenden Erfindung lag weiterhin die Aufgabe zugrunde, zur Vermeidung einer Anreicherung von Wasser im Synthesekreislauf eine gezielte Abtrennung von Wasser zu ermöglichen und eine Isolierung von 3-n-Butoxybuten-1-freiem 1-n-Butoxybuten-2 aus 3-n-Butoxybuten-1/1-n-Butoxybuten-2-haltigen Gemischen zu realisieren.

Demgemäß wurde ein Verfahren zur Abtrennung von 1,3-Butadien und Wasser aus Stoffgemischen im wesentlichen enthaltend 1,3-Butadien, 1-n-Butoxybuten-2, 3-n-Butoxybuten-1, n-Butanol, Di-n-butylether und Wasser, die bei der säurekatalysierten Umsetzung von 1,3-Butadien mit n-Butanol anfallen, gefunden, das dadurch gekennzeichnet ist, daß die 1,3-Butadien- und Wasserabtrennung durch Destillation erfolgt.

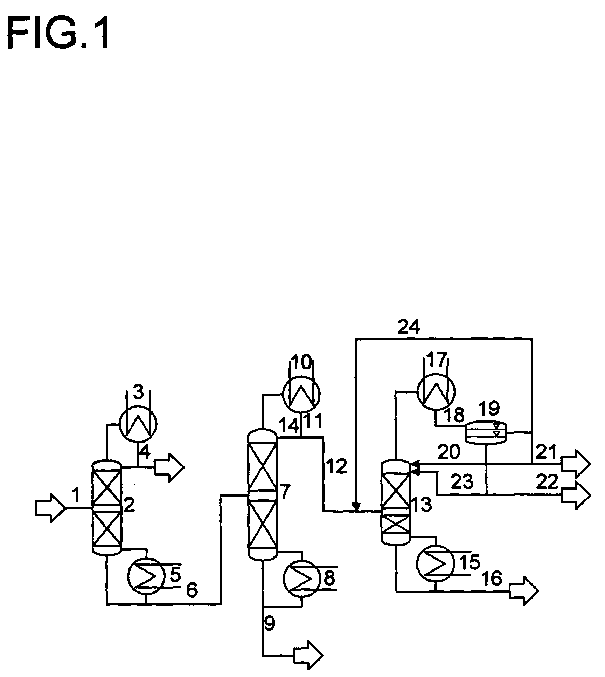

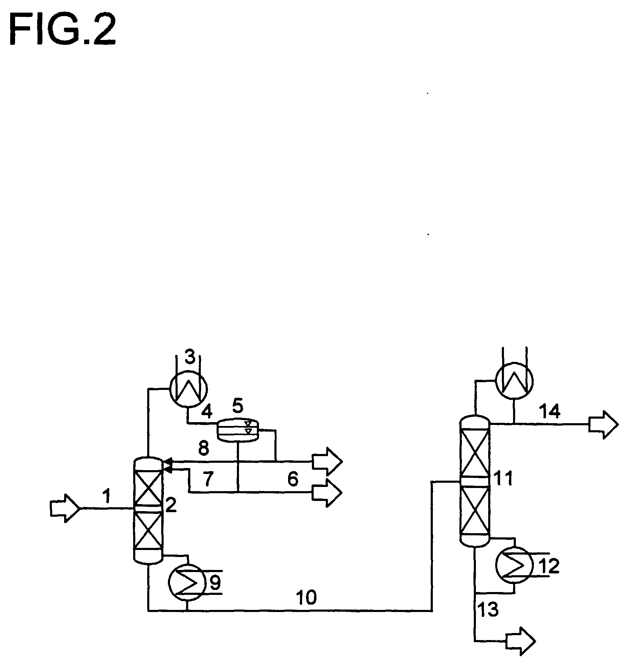

Diese Verfahrensweise kann beispielsweise wie in den Figuren 1 und 2 schematisch dargestellt realisiert werden.

Nach dem in Figur 1 beschriebenen Verfahren wird einer Destillationskolonne (2) mittels Leitung (1) ein Gemisch 1 zugeführt, welches im wesentlichen 1,3-Butadien, 1-n-Butoxybuten-2, 3-n-Butoxybuten-1, n-Butanol, Di-n-butylether und Wasser enthält. Für das erfindungsgemäße Verfahren eignen sich insbesondere Gemische, welche 1 bis 30 Gew.-%, bevorzugt 5 bis 20 Gew.-% und besonders bevorzugt 7 bis 15 Gew.-% 1,3-Butadien, 4 bis 40 Gew.-%, bevorzugt 6 bis 35 Gew.-% und besonders bevorzugt 8 bis 30 Gew.-% 1-n-Butoxybuten-2, 2 bis 50 Gew.-%, bevorzugt 10 bis 35 Gew.-% und besonders bevorzugt 15 bis 30 Gew.-% 3-n-Butoxybuten-1, 15 bis 70 Gew.-%, bevorzugt 25 bis 60 Gew.-% und besonders bevorzugt 35 bis 55 Gew.-% n-Butanol und 0 bis 10 Gew.-%, bevorzugt 0,1 bis 3 Gew.-% und besonders bevorzugt 0,4 bis 1,5 Gew.-% Wasser enthalten.

Aufgabe von Kolonne (2) ist die Abtrennung von 1,3-Butadien. Für die Trennung eignen sich allgemein übliche Kolonnen, wie beispielsweise Bodenkolonnen, Füllkörperkolonnen und Packungskolonnen. Es sind übliche Einbauten wie handelsübliche Böden, Füllkörper oder Packungen, wie beispielsweise Glockenböden, Tunnelböden, Ventilböden, Siebböden, Dualflowböden und Gitterböden, Pall-Ringe®, Berl®-Sattelkörper, Netzdrahtringe, Raschig-Ringe®, Intalox®-Sättel, Interpak®-Füllkörper und Intos®-Ringe aber auch geordnete Packungen, wie beispielsweise Sulzer-Mellapak® oder Sulzer-Optiflow®, Kühni-Rombopak® und Montz-Pak® sowie Gewebepackungen verwendbar. Die theoretische Stufenzahl der Kolonne beträgt 3 bis 30 Stufen, bevorzugt 5 bis 20 Stufen und besonders bevorzugt 7 bis 15 Stufen. Die Dimensionierung der Kolonne richtet sich nach den jeweils angestrebten Durchsätzen und kann nach den gängigen Regeln der Technik vom Fachmann problemlos durchgeführt werden.

Am Kopf der Kolonne (2) wird über einen innen- oder außenliegenden Kondensator (3) und Leitung (4) ein Produktstrom abgezogen, welcher in Abhängigkeit von der Fahrweise der Kolonne vorteilhafterweise annähernd die azeotrope Zusammensetzung aus 1,3-Butadien und Wasser aufweist. Dabei wird der Kolonnendruck üblicherweise so gewählt, daß das Butadien/Wasser-Azeotrop am Kondensator mittels Flußwasserkühlung kondensiert werden kann. In der folgenden Tabelle sind für einige Temperaturwerte die entsprechenden Butadien/Wasser-Azeotrope angegeben.

| | Azeotrope Dampfzusammensetzung | |
|---|---|---|
| Temperatur in °C | Wasser in Mol-% | 1,3-Butadien in Mol-% |
| 20 | 0,89 | 99,11 |
| 40 | 1,65 | 98,35 |
| 60 | 2,65 | 97,35 |

Das in flüssiger Form anfallende Butadien/Wasser-Azeotrop kann direkt wieder in die Umsetzungsstufe von 1,3-Butadien mit n-Butanol gemäß dem in D1 beschriebenen Verfahren eingeschleust werden. Ein Teil des Butadien/Wasser-Azeotrops kann aber auch der Kolonne (2) am Kolonnenkopf als Rücklauf und/oder als Quenchflüssigkeit zugeführt werden. Um eine Polymerisation des abdestillierten 1,3-Butadiens zu vermeiden, kann ein Polymerisationsinhibitor, wie beispielsweise das marktübliche p-tert.-Butylbrenzcatechin zudosiert werden. Die Menge an Polymerisationsinhibitor liegt üblicherweise im Bereich zwischen 50 bis 1000 ppm.

Selbstverständlich ist es auch möglich, sich im Reaktionskreislauf aufpegelnde Leichtsieder, wie beispielsweise Butane und Butene als begleitende Verunreinigungen des Butadiens, nach dem Kondensator (3) aus dem Synthesekreislauf auszuschleusen.

Die Kolonne (2) wird üblicherweise bei einem absoluten Druck am Kolonnenkopf von etwa 1 bis 10 bar, bevorzugt 2 bis 7 bar und besonders bevorzugt bei 3 bis 6 bar betrieben. Die Temperaturen in der Kolonne liegen im besonders bevorzugten Bereich bei etwa 25 bis 55°C am Kolonnenkopf und bei 150 bis 180°C am Kolonnensumpf. Die Kolonne wird mit einem Rücklaufverhältnis von 0,5 bis 10, bevorzugt 0,7 bis 2 und besonders bevorzugt von 0,8 bis 1,2 betrieben.

Am Sumpf der Kolonne (2), welcher in üblicher Art und Weise mit einem Verdampfer (5) verbunden ist, wird mittels Leitung (6) ein Reaktionsgemisch abgezogen, dessen Restgehalt an 1,3-Butadien < 5000 ppm, bevorzugt < 1000 ppm und besonders bevorzugt < 100 ppm beträgt. Dieses Reaktionsgemisch, das neben den Hauptkomponenten n-Butanol, 1-n-Butoxybuten-2 und 3-n-Butoxybuten-1 im wesentlichen noch Di-n-butylether und Spuren von Wasser enthält, wird einer Destillationskolonne (7) zwischen Verstärkungs- und Abtriebsteil zugeführt.

Aufgabe der Kolonne (7) ist die Trennung der beiden Isomeren 1-n-Butoxybuten-2 und 3-n-Butoxybuten-1. Für die Trennung eignen sich allgemein übliche Kolonnen, wie beispielsweise Bodenkolonnen, Füllkörperkolonnen und Packungskolonnen. Es sind übliche Einbauten wie handelsübliche Böden, Füllkörper oder Packungen, wie beispielsweise Glockenböden, Tunnelböden, Ventilböden, Siebböden, Dualflowböden und Gitterböden, Pall-Ringe®, Berl®-Sattelkörper, Netzdrahtringe, Raschig-Ringe®, Intalox®-Sättel, Interpak®-Füllkörper und Intos®-Ringe aber auch geordnete Packungen, wie beispielsweise Sulzer-Mellapak® oder Sulzer-Optiflow®, Kühni-Rombopak® und Montz-Pak® sowie Gewebepackungen verwendbar. Die theoretische Stufenzahl der Kolonne beträgt 10 bis 70 Stufen, bevorzugt 15 bis 50 Stufen und besonders bevorzugt 25 bis 40 Stufen. Die Dimensionierung der Kolonne richtet sich nach den jeweils angestrebten Durchsätzen und kann nach den gängigen Regeln der Technik vom Fachmann problemlos durchgeführt werden.

Die Destillationsbedingungen werden so gewählt, daß am Sumpf, welcher in üblicher Art und Weise mit einem Verdampfer (8) verbunden ist, mittels Leitung (9) ein 1-n-Butoxybuten-2-haltiger Strom abgezogen werden kann, dessen Restgehalt an 3-n-Butoxybuten-1 < 1 Gew.-%, bevorzugt < 1000 ppm und besonders bevorzugt < 200 ppm beträgt.

Die Kolonne (7) wird üblicherweise bei einem absoluten Druck am Kolonnenkopf von etwa 0,2 bis 5 bar, bevorzugt 0,5 bis 2 bar und besonders bevorzugt bei 0,8 bis 1,2 bar und ganz besonders bevorzugt bei 1 bar betrieben. Die Temperaturen in der Kolonne liegen bei etwa 50 bis 170°C am Kolonnenkopf und bei 90 bis 220°C am Kolonnensumpf. Die Kolonne wird vorteilhaft mit einem Rücklaufverhältnis von 0,5 bis 10, bevorzugt 0.7 bis 3 und besonders bevorzugt von 0,8 bis 1,8 betrieben.

Der Betrieb der Kolonne (7) ist durch zwei Betriebszustände charakterisiert. Ursache hierfür ist die Bildung eines binären Azeotrops 2, bestehend aus n-Butanol und 3-n-Butoxybuten-1, das abhängig vom Druck verschiedene Zusammensetzungen und Siedetemperaturen aufweist, wie sie beispielsweise in folgender Tabelle wiedergegeben sind:

| Druck in bar (absolut) | 1 | 5 |
|---|---|---|
| n-Butanol in Gew.-% | 50,0 | 81,1 |
| 3-n-Butoxybuten-1 in Gew.-% | 50,0 | 18,9 |
| Siedetemperatur in °C | 115 | 171 |

Enthält das der Destillationskolonne (7) aus Kolonne (2) zugeführte Gemisch mehr 3-n-Butoxybuten-1, als über das bei Destillationsbedingungen mit vorhandenem n-Butanol sich bildendem Azeotrop 2 azeotrop abdestilliert werden kann, fällt am Kopf der Kolonne (7) ein Destillat an, das aus dein Azeotrop 2, 3-n-Butoxybuten-1 und kleinen Mengen an Wasser besteht. Im Kolonnensumpf kann dann über Leitung (9) ein 3-n-Butoxybuten-1-freier 1-n-Butoxybuten-2-Strom abgetrennt und den in D1 beschriebenen möglichen Umsetzungen zu n-Butyraldehyd und/oder n-Butanol zugeführt werden. Ebenfalls in diesem Sumpfstrom enthalten sind alle aus dem in D1 beschriebenen Verfahren bedingten Hochsieder, wie beispielsweise Di-n-butylether, aber auch Di- oder Oligomere des Butadiens und deren Butoxyaddukte.

Enthält das der Destillationskolonne (7) aus Kolonne (2) zugeführte Gemisch jedoch mehr n-Butanol, als durch die destillative Abtrennung von 3-n-Butoxybuten-1, über das sich bei Destillationsbedingungen mit n-Butanol bildende Azeotrop 2 abdestilliert werden kann, werden die Destillationsbedingungen so gewählt, daß am Kopf der Kolonne ein Destillat anfällt, das aus dem Azeotrop 2, n-Butanol und kleinen Mengen an Wasser besteht. Im Sumpf kann über Leitung (9) ein 3-n-Butoxybuten-1-freier 1-n-Butoxybuten-2-Strom ausgeschleust und den in D1 beschriebenen möglichen Umsetzungen zu n-Butyraldehyd und/oder n-Butanol zugeführt werden. Ebenfalls in diesem Sumpfstrom enthalten sind alle aus dem in D1 beschriebenen Verfahren bedingten Hochsieder.

Ist es aus Gründen, die nicht Bestandteil dieser Erfindung sind, erforderlich, daß auch der Sumpfaustrag n-Butanol enthält, so ist dies prinzipiell durch geeignete Variation der Destillationsparameter möglich.

Die Aufarbeitung der Sumpfströme aus Leitung (9) und deren destillative Auftrennung in die verschiedenen Komponenten, erfolgt in der Regel nach der Umsetzung von 1-n-Butoxybuten-2 zum n-Butyraldehyd und/oder n-Butanol in üblicher Weise.

Am Kopf der Kolonne (7) wird über einen innen- oder außenliegenden Kondensator (10) und Leitung (11) ein Produktstrom abgezogen, der in der Regel 3-n-Butoxybuten-1, n-Butanol und Wasser enthält. Das nach dem Kondensationsschritt in flüssiger Form vorliegende Kondensat kann über Leitung (12) einer weiteren Kolonne (13) zugeführt werden. Ein Teil des flüssigen Destillats kann der Kolonne (7) aber auch am Kolonnenkopf als Quenchflüssigkeit oder über Leitung (14) als Rücklauf zugeführt werden.

Aufgabe der Kolonne (13) ist die Abtrennung und Ausschleusung von Wasser aus den in Kolonne (7) als Destillat anfallenden Gemischen, die im wesentlichen aus 3-n-Butoxybuten-1, n-Butanol und Wasser bestehen. Für die Trennung eignen sich allgemein übliche Kolonnen, wie beispielsweise Bodenkolonnen, Füllkörperkolonnen und Packungskolonnen. Es sind übliche Einbauten wie handelsübliche Böden, Füllkörper oder Packungen, wie beispielsweise Glockenböden, Tunnelböden, Ventilböden, Siebböden, Dualflowböden und Gitterböden, Pall-Ringe®, Berl®-Sattelkörper, Netzdrahtringe, Raschig-Ringe®, Intalox®-Sättel, Interpak®-Füllkörper und Intos®-Ringe aber auch geordnete Packungen, wie beispielsweise Sulzer-Mellapak® oder Sulzer-Optiflow®, Kühni-Rombopak® und Montz-Pak® sowie Gewebepackungen verwendbar. Die theoretische Stufenzahl der Kolonne beträgt 1 bis 30 Stufen, bevorzugt 2 bis 20 Stufen und besonders bevorzugt 3 bis 10 Stufen. Die Dimensionierung der Kolonne richtet sich nach den jeweils angestrebten Durchsätzen und kann nach den gängigen Regeln der Technik vom Fachmann problemlos ermittelt werden.

Die Kolonne (13) wird üblicherweise bei einem absoluten Druck am Kolonnenkopf von etwa 0,2 bis 5 bar, bevorzugt 0,5 bis 2 bar und besonders bevorzugt bei 0,8 bis 1,2 bar und ganz besonders bevorzugt bei 1 bar betrieben. Die Temperaturen in der Kolonne liegen bei etwa 50 bis 150°C am Kolonnenkopf und bei 50 bis 170°C am Kolonnensumpf.

Die Kolonne (13) wird im Regelfall so betrieben, daß am Kolonnensumpf, welcher in üblicher Art und Weise mit einem Verdampfer (15) verbunden ist, mittels Leitung (16) im wesentlichen ein Gemisch aus 3-n-Butoxybuten-1 und n-Butanol abgezogen werden kann. Es ist möglich, diesen Strom direkt der in D1 beschriebenen Isomerisierungs-/Synthesestufe zuzuführen. Sollte es aus Gründen des in D1 beschriebenen Verfahrens erforderlich sein, im Sumpfaustrag der Kolonne (13) einen bestimmten Wassergehalt einzustellen, so ist dies prinzipiell durch die geeignete Wahl der Destillationsbedingungen, wie beispielsweise Energieeintrag, oder Rücklaufverhältnis möglich.

Die Betriebsbedingungen der Kolonne (13) werden üblicherweise so gewählt, daß am Kolonnenkopf ein dampfförmiges Gemisch, bestehend im wesentlichen aus Wasser, 3-n-Butoxybuten-1 und n-Butanol anfällt. Dieses dampfförmige Gemisch wird dem innen- oder außenliegenden Kondensator (17) zugeführt, dort kondensiert und das anfallende flüssige Kondensat über Leitung (18) in einen Phasenscheider (19) geführt, in dem sich eine organische Phase, bestehend im wesentlichen aus 3-n-Butoxybuten-1, und n-Butanol sowie kleinen Mengen Wasser sowie eine wäßrige Phase, bestehend im wesentlichen aus Wasser sowie kleinen Mengen von n-Butanol und Spuren von 3-n-Butoxybuten-1 und 1-n-Butoxybuten-2, ausbildet.

Als Phasenabscheider eignen sich z.B. Schwerkraftphasenscheider, die ohne oder mit Einbauten, wie beispielsweise Gestricken, Füllkörpern oder Umlenkblechen ausgestattet sind oder auch Zentrifugalabscheider.

Die sich im Phasenabscheider (19) ausbildende organische Phase wird in der Regel der Kolonne (13) über Leitung (20) am Kopf der Kolonne als Rücklauf zugeführt. Prinzipiell ist es aber auch möglich, einen Teil der organischen Phase über Leitung (24) auf die Zulaufleitung (12) zur Kolonne (13) zu führen oder über Leitung (21) abzutrennen.

Die sich im Phasenabscheider (19) ausbildende wäßrige Phase wird üblicherweise über Leitung (22) abgetrennt. Prinzipiell ist es aber auch möglich, einen Teil der wäßrigen Phase über Leitung (23) am Kopf der Kolonne (13) zurückzufahren.

Nach dem in Figur 2 beschriebenen Verfahren wird der Destillationskolonne (2) mittels Leitung (1) ein Gemisch 1 zugeführt.

Aufgabe der Kolonne (2) ist die Abtrennung von 1,3-Butadien und Wasser. Für die Trennung eignen sich allgemein übliche Kolonnen, wie beispielsweise Bodenkolonnen, Füllkörperkolonnen und Packungskolonnen. Es sind übliche Einbauten wie handelsübliche Böden, Füllkörper oder Packungen, wie beispielsweise Glockenböden, Tunnelböden, Ventilböden, Siebböden, Dualflowböden und Gitterböden, Pall-Ringe®, Berl®-Sattelkörper, Netzdrahtringe, Raschig-Ringe®, Intalox®-Sättel, Interpak®-Füllkörper und Intos®-Ringe aber auch geordnete Packungen, wie beispielsweise Sulzer-Mellapak® oder Sulzer-Optiflow®, Kühni-Rombopak® und Montz-Pak® sowie Gewebepackungen verwendbar. Die theoretische Stufenzahl der Kolonne beträgt 3 bis 30 Stufen, bevorzugt 5 bis 20 Stufen und besonders bevorzugt 7 bis 15 Stufen. Die Dimensionierung der Kolonne richtet sich nach den jeweils angestrebten Durchsätzen und kann nach den gängigen Regeln der Technik vom Fachmann problemlos durchgeführt werden.

Am Kopf der Kolonne wird über einen innen- oder außenliegenden Kondensator (3) und Leitung (4) ein Produktstrom abgezogen, welcher in Abhängigkeit von der Fahrweise der Kolonne vorteilhafterweise annähernd die azeotrope Zusammensetzung aus 1,3-Butadien und Wasser aufweist. Dabei wird der Kolonnendruck üblicherweise so gewählt, daß das Butadien/Wasser-Azeotrop am Kondensator mittels Flußwasserkühlung kondensiert werden kann.

Das in flüssiger Form anfallende Butadien/Wasser-Azeotrop wird vorteilhaft in einen Phasenabscheider (5) geleitet, in dem sich eine organische Phase, die im wesentlichen 1,3-Butadien enthält und eine wäßrige Phase ausbilden.

Als Phasenabscheider eignen sich z.B. Schwerkraftphasenscheider, die ohne oder mit Einbauten, wie beispielsweise Gestricken, Füllkörpern oder Umlenkblechen ausgestattet sind oder wie auch Zentrifugalabscheider.

Die wäßrige Phase wird üblicherweise über Leitung (6) abgetrennt. Es ist aber auch prinzipiell möglich, einen Teil der wäßrigen Phase über Leitung (7) am Kopf der Kolonne als Rücklauf zuzuführen.

Das in der organischen Phase vorliegende 1,3-Butadien kann prinzipiell direkt wieder in die Umsetzungsstufe von 1,3-Butadien mit n-Butanol gemäß D1 eingeschleust werden. Es ist aber auch möglich, einen Teil des 1,3-Butadiens der Kolonne (2) am Kolonnenkopf als Quenchflüssigkeit oder über Leitung (8) als Rücklauf zuzuführen. Üblicherweise werden Teilmengen des 1,3-Butadiens sowohl der Umsetzungsstufe mit n-Butanol, als auch dem Rücklauf und/oder dem Quench zugeführt. Um eine Polymerisation des abdestillierten 1,3-Butadiens zu vermeiden, kann ein Polymerisationsinhibitor, wie beispielsweise das marktübliche p-tert.-Butylbrenzcatechin zudosiert werden. Die Menge an Polymerisationsinhibitor liegt üblicherweise im Bereich zwischen 50 bis 1000 ppm.

Selbstverständlich ist es auch möglich, sich im Reaktionssystem aufpegelnde Leichtsieder, wie beispielsweise Butane und Butene als begleitende Verunreinigungen des Butadiens, nach dem Phasenabscheider (5) aus dem Synthesekreislauf auszuschleusen.

Die Kolonne (2) wird üblicherweise bei einem absoluten Druck am Kolonnenkopf von etwa 1 bis 10 bar, bevorzugt 2 bis 7 bar und besonders bevorzugt bei 3 bis 6 bar betrieben. Die Temperaturen in der Kolonne liegen im besonders bevorzugten Bereich bei etwa 25 bis 55°C am Kolonnenkopf und bei 150 bis 180°C am Kolonnensumpf. Die Kolonne wird vorteilhaft mit einem Rücklaufverhältnis von 0,5 bis 10, bevorzugt 0,7 bis 2 und besonders bevorzugt von 0,8 bis 1,2 betrieben.

Am Sumpf der Kolonne (2), welcher in üblicher Art und Weise mit einem Verdampfer (9) verbunden ist, wird mittels Leitung (10) ein Reaktionsgemisch 3 abgezogen, dessen Restgehalt an 1,3-Butadien < 5000 ppm, bevorzugt < 1000 ppm und besonders bevorzugt < 100 ppm beträgt. Dieses Reaktionsgemisch, das im wesentlichen 1-n-Butoxybuten-2, 3-n-Butoxybuten-1, n-Butanol sowie Spuren von Wasser und Hochsiedern, wie beispielsweise Di-n-butylether enthält, wird einer Destillationskolonne (11) zwischen Verstärkungs- und Abtriebsteil zugeführt.

Aufgabe der Kolonne (11) ist die Trennung der beiden Isomeren 1-n-Butoxybuten-2 und 3-n-Butoxybuten-1. Für die Trennung eignen sich allgemein übliche Kolonnen, wie beispielsweise Bodenkolonnen, Füllkörperkolonnen und Packungskolonnen. Es sind übliche Einbauten wie handelsübliche Böden, Füllkörper oder Packungen, wie beispielsweise Glockenböden, Tunnelböden, Ventilböden, Siebböden, Dualflowböden und Gitterböden, Pall-Ringe®, Berl®-Sattelkörper, Netzdrahtringe, Raschig-Ringe®, Intalox®-Sättel, Interpak®-Füllkörper und Intos®-Ringe aber auch geordnete Packungen, wie beispielsweise Sulzer-Mellapak® oder Sulzer-Optiflow®, Kühni-Rombopak® und Montz-Pak® sowie Gewebepackungen verwendbar. Die theoretische Stufenzahl der Kolonne beträgt 10 bis 70 Stufen, bevorzugt 15 bis 50 Stufen und besonders bevorzugt 25 bis 40 Stufen. Die Dimensionierung der Kolonne richtet sich nach den jeweils angestrebten Durchsätzen und kann nach den gängigen Regeln der Technik vom Fachmann problemlos ermittelt werden.

Die Destillationsbedingungen werden so gewählt, daß am Sumpf, welcher in üblicher Art und Weise mit einem Verdampfer (12) verbunden ist, mittels Leitung (13) 1-n-Butoxybuten-2-haltiger Strom abgezogen werden kann, dessen Restgehalt an 3-n-Butoxybuten-1 < 1 Gew.-%, bevorzugt < 1000 ppm und besonders bevorzugt < 200 ppm beträgt.

Für den Betrieb der Kolonne (11) sind zwei prinzipielle Betriebszustände denkbar. Ursache hierfür ist die Bildung des binären Azeotrops 2.

Enthält das der Destillationskolonne (11) aus Kolonne (2) zugeführte Gemisch mehr 3-n-Butoxybuten-1, als über das bei Destillationsbedingungen mit vorhandenem n-Butanol sich bildendem Azeotrop 2 azeotrop abdestilliert werden kann, fällt am Kopf der Kolonne ein Destillat an, das aus dein Azeotrop 2, 3-n-Butoxybuten-1 und Spuren von Wasser besteht und über Leitung (14) abgetrennt wird. Im Kolonnensumpf kann dann über Leitung (13) ein 3-n-Butoxybuten-1-freier 1-n-Butoxybuten-2-Strom abgetrennt werden. Der abgetrennte Strom kann prinzipiell einer in D1 beschriebenen Umsetzung zu n-Butyraldehyd und/oder n-Butanol zugeführt werden. Ebenfalls in diesem Sumpfstrom enthalten sind alle aus dem in D1 beschriebenen Verfahren bedingten Hochsieder, wie beispielsweise Di-n-butylether, aber auch Di- oder Oligomere des Butadiens und deren Butoxyaddukte.

Enthält das der Destillationskolonne (11) aus Kolonne (2) zugeführte Gemisch jedoch mehr n-Butanol, als durch die destillative Abtrennung von 3-n-Butoxybuten-1, über das sich bei Destillationsbedingungen mit n-Butanol bildende Azeotrop 2 azeotrop abdestilliert werden kann, werden die Destillationsbedingungen so gewählt, daß am Kopf der Kolonne ein Destillat anfällt, das im wesentlichen aus dem Azeotrop 2, n-Butanol und Spuren von Wasser besteht und über Leitung (14) abgetrennt wird. Im Sumpf kann dann über Leitung (13) im wesentlichen ein 3-n-Butoxybuten-1-freier 1-n-Butoxybuten-2-Strom ausgeschleust und den in D1 beschriebenen möglichen Umsetzungen zu n-Butyraldehyd und/oder n-Butanol zugeführt werden. Ebenfalls in diesem Sumpfstrom enthalten sind alle aus dem in D1 beschriebenen Verfahren bedingten Hochsieder.

Durch geeignete Variation der Destillationsparameter ist es prinzipiell auch möglich, die Abtrennung so zu steuern, daß auch der Sumpfaustrag n-Butanol enthält.

Die Aufarbeitung der Sumpfströme aus Leitung (13) und deren destillative Auftrennung in die verschiedenen Komponenten, erfolgt in der Regel nach der Umsetzung von 1-n-Butoxybuten-2 zum n-Butyraldehyd und/oder n-Butanol in allgemein bekannter Weise.

Die Kolonne (11) wird üblicherweise bei einem absoluten Druck am Kolonnenkopf von etwa 0,2 bis 5 bar, bevorzugt 0,5 bis 2 bar und besonders bevorzugt bei 0,8 bis 1,2 bar und ganz besonders bevorzugt bei 1 bar betrieben. Die Temperaturen in der Kolonne liegen bei etwa 50 bis 170°C am Kolonnenkopf und bei 90 bis 220°C am Kolonnensumpf. Die Kolonne wird üblicherweise mit einem Rücklaufverhältnis von 0,5 bis 10, bevorzugt 0,7 bis 3 und besonders bevorzugt von 0,8 bis 1,8 betrieben.

Wird gemäß des in Figur 2 beschriebenen Verfahrens der Kolonne (2) über Leitung (1) jedoch ein Stoffgemisch 1 zugeführt, aus dem mehr Wasser abdestilliert werden soll, als durch die destillative Abtrennung von Wasser, über das sich bei Destillationsbedingungen mit 1,3-Butadien bildende Azeotrop 1,3-Butadien/Wasser azeotrop abdestilliert werden kann, werden die Destillationsbedingungen so gewählt, daß am Kopf der Kolonne über einen innen- oder außenliegenden Kondensator (3) und Leitung (4) ein Produktstrom anfällt, welcher im wesentlichen 1,3-Butadien, aber auch n-Butanol, 3-n-Butoxybuten-1 und Wasser enthält. Der Kolonnendruck wird üblicherweise so gewählt, daß das anfallende dampfförmige Gemisch am Kondensator (3) mittels Flußwasserkühlung kondensiert werden kann.

Die Destillationsbedingungen werden so gewählt, daß am Sumpf der Kolonne (2), welcher in üblicher Art und Weise mit einem Verdampfer (9) verbunden ist, mittels Leitung (10) ein Reaktionsgemisch abgezogen werden kann, dessen Restgehalt an 1,3-Butadien < 5000 ppm, bevorzugt < 1000 ppm und besonders bevorzugt < 100 ppm beträgt.

Das nach Kondensation in flüssiger Form anfallende Gemisch, enthaltend im wesentlichen 1,3-Butadien, aber auch n-Butanol, 3-n-Butoxybuten-1 und Wasser wird vorteilhaft über Leitung (4) in einen Phasenabscheider (5) geleitet, in dem sich eine organische Phase, die im wesentlichen 1,3-Butadien, 3-n-Butoxybuten-1, n-Butanol sowie Spuren von Wasser enthält und eine wäßrige Phase, die im wesentlichen aus Wasser und n-Butanol besteht, ausbilden.

Als Phasenabscheider eignen sich z.B. Schwerkraftphasenscheider, die ohne oder mit Einbauten, wie beispielsweise Gestricken, Füllkörpern, oder Umlenkblechen ausgestattet sind oder auch Zentrifugalabscheider.

Die wäßrige Phase wird üblicherweise über Leitung (6) abgetrennt. Es ist aber auch prinzipiell möglich, einen Teil der wäßrigen Phase über Leitung (7) am Kopf der Kolonne (2) als Rücklauf zuzuführen.

Die organische Phase, enthaltend im wesentlichen 1,3-Butadien, aber auch n-Butanol, 3-n-Butoxybuten-1 und Wasser kann prinzipiell direkt wieder in die Umsetzungsstufe von 1,3-Butadien mit n-Butanol gemäß der Patentschrift D1 zugeführt werden. Es ist aber auch möglich, einen Teil der organischen Phase der Kolonne (2) am Kolonnenkopf als Quenchflüssigkeit oder über Leitung (8) als Rücklauf zuzuführen. Üblicherweise werden Teilmengen der organischen Phase sowohl der Umsetzungsstufe mit n-Butanol, als auch dem Rücklauf und/oder dem Quench der Kolonne zugeführt. Um eine Polymerisation des in der organischen Phase als Hauptkomponente vorliegenden 1,3-Butadiens zu vermeiden, kann ein Polymerisationsinhibitor, wie beispielsweise das marktübliche p-tert.-Butylbrenzcatechin zudosiert werden. Die Menge an Polymerisationsinhibitor liegt üblicherweise im Bereich zwischen 50 bis 1000 ppm.

Selbstverständlich ist es auch möglich, sich im Reaktionssystem anreichernde Leichtsieder, wie beispielsweise Butane und Butene als begleitende Verunreinigungen des Butadiens, nach dem Phasenabscheider (5) aus dem Synthesekreislauf auszuschleusen.

Für die Trennung eignen sich allgemein übliche Kolonnen, wie beispielsweise Bodenkolonnen, Füllkörperkolonnen und Packungskolonnen. Es sind übliche Einbauten wie handelsübliche Böden, Füllkörper oder Packungen, wie beispielsweise Glockenböden, Tunnelböden, Ventilböden, Siebböden, Dualflowböden und Gitterböden, Pall-Ringe®, Berl®-Sattelkörper, Netzdrahtringe, Raschig-Ringe®, Intalox®-Sättel, Interpak®-Füllkörper und Intos®-Ringe aber auch geordnete Packungen, wie beispielsweise Sulzer-Mellapak® oder Sulzer-Optiflow®, Kühni-Rombopak® und Montz-Pak® sowie Gewebepackungen verwendbar. Die theoretische Stufenzahl der Kolonne (2) beträgt 3 bis 30 Stufen, bevorzugt 5 bis 20 Stufen und besonders bevorzugt 7 bis 15 Stufen. Die Dimensionierung der Kolonne richtet sich nach den jeweils angestrebten Durchsätzen und kann nach den gängigen Regeln der Technik vom Fachmann problemlos durchgeführt werden.

Die Kolonne (2) wird üblicherweise bei einem absoluten Druck am Kolonnenkopf von etwa 1 bis 10 bar, bevorzugt 2 bis 7 bar und besonders bevorzugt bei 3 bis 6 bar betrieben. Die Temperaturen in der Kolonne liegen im besonders bevorzugten Bereich bei etwa 25 bis 155°C am Kolonnenkopf und bei 150 bis 180°C am Kolonnensumpf. Die Kolonne wird vorteilhaft mit einem Rücklaufverhältnis von 0,5 bis 10, bevorzugt 0,7 bis 2 und besonders bevorzugt von 0,8 bis 1,2 betrieben.

Die weitere Aufarbeitung des aus Kolonne (2) über Leitung (10) ausgeschleusten Reaktionsgemisches erfolgt in der Art und Weise, wie für das Reaktionsgemisch 3 bereits beschrieben.

### Beispiele

### Beispiel 1

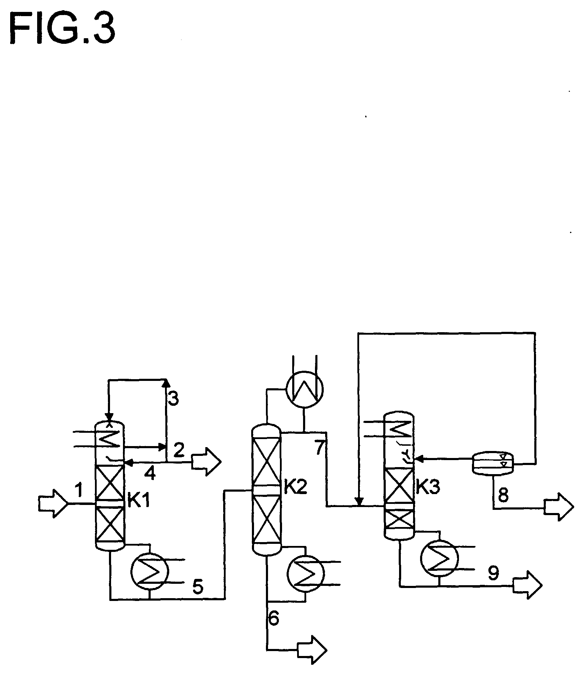

Beispiel 1 wurde in den Versuchskolonnen durchgeführt, deren schematische Anordnung in Figur 3 dargestellt ist.

Gemisch 1 wurde der Kolonne K 1 als Strom 1 zugeführt. Die Kolonne wies einen Innendurchmesser von 55 mm auf. Der Verstärkungsteil verfügte über eine Packungshöhe von 680 mm, der Abtriebsteil über 1360 mm. Die Edelstahlkolonne war mit einer geordneten Packung der Firma Sulzer ( Sulzer BX® ) ausgestattet. Der Betrieb der Kolonne erfolgte bei einem Kopfdruck von 5,5 bar (absolut). Die Sumpftemperatur betrug ca. 178°C und die Kopftemperatur ca. 37°C. Der Quenchkreis, Strom 3, am Kopf der Kolonne K 1 wurde mit 6 kg/h betrieben. Der Kolonnenrücklauf wurde durch Strom 4 realisiert. Die Abtrennung von 1,3-Butadien erfolgte über Strom 2.

Der Sumpfaustrag der Kolonne K 1 wurde über Strom 5 in die Kolonne K 2 geleitet. Die Glaskolonne K 2 wies einen Innendurchmesser von 50 mm aus und war mit einer strukturierten Packung der Firma Sulzer ( Sulzer CY® ) ausgestattet. Sie verfügte im Verstärkungsteil über eine Packungshöhe von 1360 mm und im Abtriebsteil über eine Packungshöhe von 2400 mm. Der Kopfdruck dieser Kolonne betrug 1 bar (absolut). Die Kopftemperatur betrug ca. 114°C und die Sumpftemperatur ca. 139°C. Es wurde ein Rücklauf von 2,7 kg/h eingestellt. Über Strom 6 erfolgte die Ausschleusung des 1-Butoxybuten-2-haltigen Stroms.

Das Kopfprodukt, Strom 7, wurde der Entwässerungskolonne K 3 zugeführt. Die bei 1 bar (absolut) betriebene Glaskolonne K 3 verfügte über einen Innendurchmesser von 50 mm und war mit einer geordneten Packung der Firma Sulzer ( Sulzer BX® ) ausgestattet. Der Verstärkungsteil der Kolonne verfügte über eine Packungshöhe von 680 mm und der Abtriebsteil über eine Packungshöhe von 1020 mm. Der Rücklauf wurde über einen magnetisch betriebenen Rücklaufteiler ( Klapptrichter ) auf ein Rücklaufverhältnis von 1:20 eingestellt. Sowohl die wäßrige als auch die organsiche Phase wurden als Rücklauf zurückgeführt sowie in den Phasenscheider geleitet. Die aus dem Phasenabscheider abgezogene organische Phase wurde wieder dem Zulauf der Kolonne zugegeben. Die wäßrige Phase, Strom 8, wurde ausgeschleust. Das 3-n-Butoxybuten-1 fiel in Strom 9 an. Die Sumpftemperatur betrug ca. 115°C.

Die entsprechenden Mengenströme und ihre Zusammensetzung sind Tabelle 1 zu entnehmen.

**Tabelle 1**

| Strom | 1 | 2 | 3 | 4 | 5 | 6 | 7 | 8 | 9 |
|---|---|---|---|---|---|---|---|---|---|
| Menge in g/h | 2537 | 140 | 6000 | 500 | 2397 | 402 | 1995 | 2 | 1993 |

| Zusammensetzung in Gew.-% | | | | | | | | | |
|---|---|---|---|---|---|---|---|---|---|
| 1,3-Butadien | 7,4 | 92,7 | 92,7 | 92,7 | 0 | 0 | 0 | 0 | 0 |
| n-Butanol | 47,5 | 0 | 0 | 0 | 51,2 | 10,4 | 59,0 | 2,8 | 59,8 |
| 3-n-Butoxybuten-1 | 30,0 | 0 | 0 | 0 | 32,6 | 0 | 39,3 | 2,5 | 38,7 |
| 1-n-Butoxybuten-2 | 12,2 | 0 | 0 | 0 | 13,2 | 78,2 | 0,2 | 0,1 | 0,1 |
| Di-n-butylether | 1,0 | 0 | 0 | 0 | 1,1 | 2,3 | 0,9 | 0 | 0,9 |
| Wasser | 0,2 | 0,1 | 0,1 | 0,1 | 0,1 | 0 | 0,1 | 94,6 | 0 |
| Leichtsieder | 0,4 | 7,2 | 7,2 | 7,2 | 0 | 0 | 0 | 0 | 0 |
| Hochsieder/Sonstige | 1,3 | 0 | 0 | 0 | 1,8 | 9,1 | 0,5 | 0 | 0,5 |

### Beispiel 2

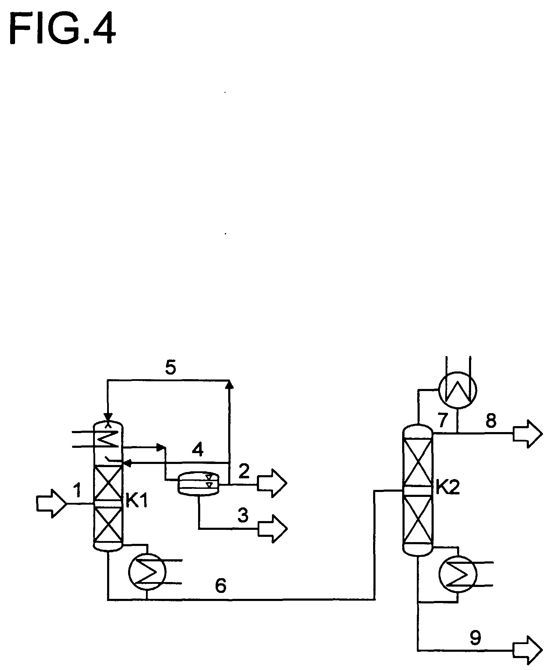

Das Beispiel 2 wurde in Versuchskolonnen durchgeführt, deren schematische Anordnung in Figur 4 dargestellt ist.

Gemisch 1 wurde der Kolonne K 1 als Strom 1 zugeführt. Die Kolonne wies einen Innendurchmesser von 55 mm auf. Der Verstärkungsteil verfügte über eine Packungshöhe von 680 mm, der Abtriebsteil über 1360 mm. Die Edelstahlkolonne war mit einer geordneten Packung der Firma Sulzer ( Sulzer BX® ) ausgestattet. Der Betrieb der Kolonne erfolgte bei einem Kopfdruck von 5,0 bar (absolut). Die Sumpftemperatur betrug ca. 166°C und die Kopftemperatur ca. 26°C. Das Kondensat leitete man in einen Phasenabscheider. Die Abtrennung von 1,3-Butadien erfolgte durch Strom 2. Der 1,3-Butadienrücklauf zur Kolonne erfolgte über Strom 4. Der Quenchkreis, Strom 5, am Kopf der Kolonne K 1 wurde mit 5,0 kg/h betrieben. Die wäßrige Phase schleuste man über Strom 3 aus.

Der Sumpfaustrag der Kolonne K 1 leitete man über Strom 6 in die Kolonne K 2. Der Kopfdruck dieser Kolonne betrug 1 bar (absolut). Die Glaskolonne K 2 wies einen Innendurchmesser von 50 mm aus und war mit einer strukturierten Packung der Firma Sulzer (Sulzer CY®) ausgestattet. Sie verfügte im Verstärkungsteil über eine Packungshöhe von 1360 mm und im Abtriebsteil über eine Packungshöhe von 2400 mm. Der Rücklauf erfolgte über Strom 7. Die Kopftemperatur betrug ca. 113°C und die Sumpftemperatur ca. 125°C. Über Strom 9 erfolgte die Ausschleusung des 1-n-Butoxybuten-2-haltigen Stroms. Das 3-n-Butoxybuten-1 wurden über Strom 8 ausgetragen.

Die entsprechenden Mengenströme und ihre Zusammensetzung sind Tabelle 2 zu entnehmen.

**Tabelle 2**

| Strom | 1 | 2 | 3 | 4 | 5 | 6 | 7 | 8 | 9 |
|---|---|---|---|---|---|---|---|---|---|
| Menge in g/h | 2040 | 197 | 2 | 500 | 5000 | 1840 | 3500 | 1470 | 370 |

| Zusammensetzung in Gew.-% | | | | | | | | | |
|---|---|---|---|---|---|---|---|---|---|
| 1,3-Butadien | 9,0 | 92,7 | 0 | 92,7 | 92,7 | 0 | 0 | 0 | 0 |
| n-Butanol | 57,6 | 0 | 0 | 0 | 0 | 63,5 | 69,5 | 69,5 | 35,5 |
| 3-n-Butoxybuten-1 | 15,3 | 0 | 0 | 0 | 0 | 16,8 | 21,4 | 21,4 | 0 |
| 1-n-Butoxybuten-2 | 10,9 | 0 | 0 | 0 | 0 | 12,2 | 1,1 | 1,1 | 59,0 |
| Di-n-butylether | 2,6 | 0 | 0 | 0 | 0 | 2,9 | 3,2 | 3,2 | 1,5 |
| Wasser | 1,0 | 0,1 | 100 | 0,1 | 0,1 | 0,9 | 1,1 | 1,1 | 0 |
| Leichtsieder | 0,6 | 7,2 | 0 | 7,2 | 7,2 | 0 | 0 | 0 | 0 |
| Hochsieder/Sonstige | 3,0 | 0 | 0 | 0 | 0 | 3,7 | 4,1 | 4,1 | 4,0 |

### Beispiel 3

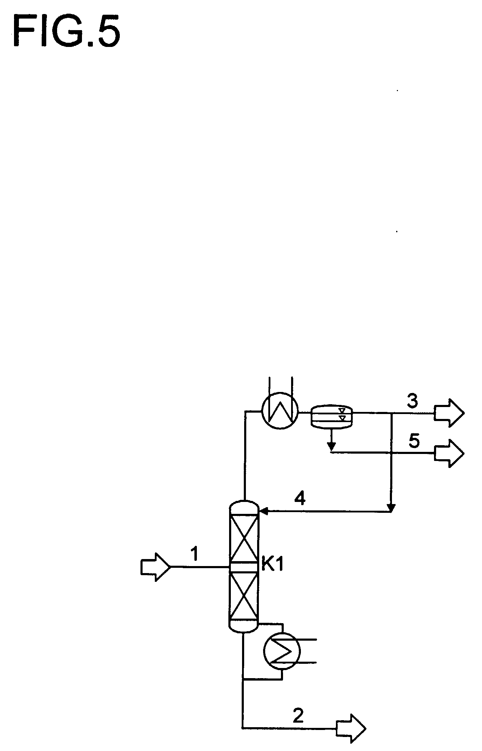

Beispiel 3 soll aufzeigen, daß ein größerer Wasserstrom abgetrennt werden kann, als dies durch die Abtrennung in Form des reinen 1,3-Butadien/Wasser-Azeotrops möglich ist. Es erfolgte daher nur die erste Trennungsstufe (Abtrennung von 1,3-Butadien und Wasser) in einer Versuchskolonne, deren schematische Anordnung in Figur 5 dargestellt ist. Auf die Trennung von 1-n-Butoxybuten-2 und 3-n-Butoxybuten-1 wurde verzichtet.

Gemisch 1 wurde der Kolonne K 1 als Strom 1 zugeführt. Die Glockenbodenkolonne aus Edelstahl wies einen Innendurchmesser von 70 mm auf. Der Verstärkungsteil verfügte über 40 Böden und der Abtriebsteil über 20 Böden. Der Betrieb der Kolonne erfolgte bei einem Kopfdruck von 5,0 bar (absolut). Die Kopftemperatur betrug 93°C und die Sumpftemperatur lag bei 176°C. Der Kopfstrom wurde kondensiert und in einen Phasenabscheider geleitet. Die dort gebildete wäßrige Phase trennte man über Strom 5 ab. Die anfallende organische Phase, enthaltend im wesentlichen 1,3-Butadien, 3-n-Butoxybuten-1 und n-Butanol wurde in Strom 3 und Strom 4 aufgeteilt. Strom 4 führte man auf den Kopf der Kolonne K 1 zurück und stellte damit ein Rücklaufverhältnis von 7 ein. Die Ausschleusung des 1,3-Butadien-haltigen Stroms erfolgte durch Strom 3.

Die Hauptmengen an 1-n-Butoxybuten-2, 3-n-Butoxybuten-1 und n-Butanol waren im Sumpfstrom 2 enthalten.

Die entsprechenden Mengenströme und ihre Zusammensetzung sind Tabelle 3 zu entnehmen.

**Tabelle 3**

| Strom | 1 | 2 | 3 | 4 | 5 |
|---|---|---|---|---|---|
| Menge in g/h | 482 | 370 | 86 | 602 | 26 |

| Zusammensetzung in Gew.-% | | | | | |
|---|---|---|---|---|---|
| 1,3-Butadien | 14,1 | 0 | 80,3 | 80,3 | 0 |
| n-Butanol | 67,1 | 85,7 | 7,7 | 7,7 | 1,6 |
| 3-n-Butoxybuten-1 | 3,9 | 2,3 | 11,8 | 11,8 | 0 |
| 1-n-Butoxybuten-2 | 8,2 | 10,6 | 0 | 0 | 0 |
| Di-n-butylether | 0,7 | 0,9 | 0 | 0 | 0 |
| Wasser | 5,5 | 0 | 0,2 | 0,2 | 98,4 |
| Hochsieder/Sonstige | 0,5 | 0,5 | 0 | 0 | 0 |

## Patentansprüche

1. Verfahren zur Abtrennung von 1,3-Butadien und Wasser aus Stoffgemischen enthaltend 1,3-Butadien, 1-n-Butoxybuten-2, 3-n-Butoxybuten-1, n-Butanol, Di-n-butylether und Wasser, die bei der säurekatalysierten Umsetzung von 1,3-Butadien mit n-Butanol anfallen, dadurch gekennzeichnet, daß die 1,3-Butadien- und Wasser-Abtrennung durch Destillation erfolgt.

2. Verfahren nach Anspruch 1, dadurch gekennzeichnet, daß man
a) das Stoffgemisch in eine erste Destillationskolonne führt, über Kopf im wesentlichen 1,3-Butadien abtrennt und daß man das aus dem Sumpf der Destillationskolonne abgezogene, im wesentlichen 1,3-Butadien-freie Gemisch
b) in eine zweite Destillationskolonne führt, am Sumpf dieser zweiten Destillationskolonne im wesentlichen einen 3-n-Butoxybuten-1-freien 1-n-Butoxybuten-2-Strom abtrennt und das am Kopf der zweiten Kolonne gebildete Gemisch
c) in eine dritte Destillationskolonne führt, im Sumpf dieser dritten Kolonne ein Gemisch im wesentlichen bestehend aus 3-n-Butoxybuten-1 und n-Butanol abtrennt, das über Kopf erhältliche heterogene Gemisch einer Phasentrennung unterzieht, die organische Phase als Rück- oder Zulauf zur dritten Kolonne einsetzt und das Wasser abtrennt.

3. Verfahren nach Anspruch 1, dadurch gekennzeichnet, daß man
a) das Stoffgemisch in eine erste Destillationskolonne führt, über Kopf im wesentlichen ein Gemisch bestehend aus 1,3-Butadien und Wasser abtrennt, und daß man das aus dem Sumpf dieser Destillationskolonne abgezogene, im wesentlichen 1,3-Butadien-freie Gemisch
b) in eine zweite Destillationskolonne führt, am Sumpf dieser zweiten Destillationskolonne im wesentlichen einen 3-n-Butoxybuten-1-freien 1-n-Butoxybuten-2-Strom und am Kopf dieser zweiten Kolonne ein Gemisch aus 3-n-Butoxybuten-1 und n-Butanol abtrennt.

4. Verfahren nach Anspruch 3, dadurch gekennzeichnet, daß man
a) das Stoffgemisch in eine erste Destillationskolonne führt, über Kopf ein heterogenes Gemisch im wesentlichen bestehend aus 1,3-Butadien, n-Butanol, 3-n-Butoxybuten-1 und Wasser abtrennt, dieses in einer Phasentrennung in eine organische Phase, enthaltend im wesentlichen 1,3-Butadien, 3-n-Butoxybuten-1, n-Butanol und Spuren von Wasser und in eine wäßrige Phase enthaltend im wesentlichen Wasser und gelöstes n-Butanol, auftrennt und daß man das aus dem Sumpf der Destillationskolonne abgezogene, im wesentlichen 1,3-Butadien-freie Gemisch
b) in eine zweite Destillationskolonne führt, am Sumpf dieser zweiten Destillationskolonne im wesentlichen einen 3-n-Butoxybuten-1-freien 1-n-Butoxybuten-2-Strom und am Kopf dieser zweiten Kolonne ein Gemisch aus 3-n-Butoxybuten-1 und n-Butanol abtrennt.

5. Verfahren nach einem der Ansprüche 2, 3 und 4 dadurch gekennzeichnet, daß der Sumpfaustrag der ersten Destillationskolonne weniger als 5000 ppm 1,3-Butadien enthält.

6. Verfahren nach einem der Ansprüche 2, 3 und 4 dadurch gekennzeichnet, daß der Sumpfaustrag der zweiten Destillationskolonne weniger als 1 Gew.-% 3-n-Butoxybuten-1 enthält.

7. Verfahren nach einem der Ansprüche 2, 3 und 4 dadurch gekennzeichnet, daß der absolute Druck in der ersten Destillationskolonne 1 bis 10 bar beträgt.

8. Verfahren nach Anspruch 2 dadurch gekennzeichnet, daß der absolute Druck in der zweiten und dritten Destillationskolonne 0,2 bis 5 bar beträgt.

9. Verfahren nach einem der Ansprüche 3 und 4 dadurch gekennzeichnet, daß der absolute Druck in der zweiten Destillationskolonne 0,2 bis 5 bar beträgt.

10. Verfahren nach Anspruch 3 dadurch gekennzeichnet, daß man das am Kopf der ersten Destillationskolonne anfallende 1,3-Butadien/Wasser-Gemisch kondensiert und einer Phasentrennung unterzieht.

11. Verfahren nach Anspruch 4 dadurch gekennzeichnet, daß man das am Kopf der ersten Destillationskolonne anfallende heterogene Gemisch kondensiert und einer Phasentrennung unterzieht.

12. Verfahren nach Anspruch 2 dadurch gekennzeichnet, daß man den am Kopf der ersten Destillationskolonne anfallenden 1,3-Butadien-haltigen Strom einer Umsetzung mit n-Butanol zu 1-n-Butoxybuten-2/3-n-Butoxybuten-1-Gemischen zuführt.

13. Verfahren nach Anspruch 10 dadurch gekennzeichnet, daß man die bei der Phasentrennung gebildete organische Phase einer Umsetzung mit n-Butanol zu 1-n-Butoxybuten-2/3-n-Butoxybuten-1-Gemischen zuführt.

14. Verfahren nach Anspruch 11 dadurch gekennzeichnet, daß man die bei der Phasentrennung gebildete organische Phase einer Umsetzung mit n-Butanol zu 1-n-Butoxybuten-2/3-n-Butoxybuten-1-Gemischen zuführt.

15. Verfahren nach einem der Ansprüche 2, 3 und 4 dadurch gekennzeichnet, daß man die 3-n-Butoxybuten-1-haltigen Ströme einer Umsetzung zu 1-n-Butoxybuten-2/3-n-Butoxybuten-1-Gemischen zuführt.

16. Verfahren nach einem der Ansprüche 2, 3 und 4 dadurch gekennzeichnet, daß man die 3-n-Butoxybuten-1-haltigen Ströme einer Isomerisierung zu 1-n-Butoxybuten-2 mit nachfolgender Umsetzung zu 1-n-Butoxybuten-2/3-n-Butoxybuten-1-Gemischen zuführt.
